# EUROPEAN PATENT APPLICATION

(11) **EP 4 233 599 A2**
(43) Date of publication of application: **30.08.2023**
(21) Application number: 23164364.4
(22) Date of filing: 17.09.2020
(51) Int. Cl.: A24F 40/50

(54) **AEROSOL PROVISION SYSTEM AND METHOD**

(30) Priority: 16.10.2019 GB 201914949
(62) Divisional of application: 20780289.3
(71) Applicant: Nicoventures Trading Limited, London WC2R 3LA (GB)
(72) Inventor: MOLONEY, Patrick, LONDON, WC2R 3LA (GB)
(74) Representative: D Young & Co LLP

(57) **Abstract**

A system, comprising an aerosol provision system configured to generate aerosol from an aerosol generating material for user inhalation, comprises a computer configured to obtain user behaviour data relating to an interaction by a user with the aerosol provision system, determine default user behaviour with respect to the interaction on the basis of the obtained user behaviour data, monitor a current interaction by the user with the aerosol provision system, and when the current user interaction deviates from the default user behaviour by a pre-determined amount, adjust the operational parameters of the aerosol provision system on the basis of the current user interaction.

## Description

### BACKGROUND OF THE DISCLOSURE

### Field

The present disclosure relates to an aerosol provision system and method.

### Description of the Prior Art

The "background" description provided herein is for the purpose of generally presenting the context of the disclosure. Work of the presently named inventors, to the extent it is described in this background section, as well as aspects of the description which may not otherwise qualify as prior art at the time of filing, are neither expressly or impliedly admitted as prior art against the present disclosure.

Electronic aerosol provision systems such as electronic cigarettes (e-cigarettes) generally contain a reservoir of a source liquid containing a formulation, typically including nicotine, from which an aerosol is generated, e.g. through heat vaporisation. An aerosol source for an aerosol provision system may thus comprise a heater having a heating element arranged to receive source liquid from the reservoir, for example through wicking / capillary action. Other source materials may be similarly heated to create an aerosol, such as botanical matter, or a gel comprising an active ingredient and/or flavouring. Hence more generally, the e-cigarette may be thought of as comprising or receiving a payload for heat vaporisation.

While a user inhales on the device, electrical power is supplied to the heating element to vaporise the aerosol source (a portion of the payload) in the vicinity of the heating element, to generate an aerosol for inhalation by the user. Such devices are usually provided with one or more air inlet holes located away from a mouthpiece end of the system. When a user sucks on a mouthpiece connected to the mouthpiece end of the system, air is drawn in through the inlet holes and past the aerosol source. There is a flow path connecting between the aerosol source and an opening in the mouthpiece so that air drawn past the aerosol source continues along the flow path to the mouthpiece opening, carrying some of the aerosol from the aerosol source with it. The aerosol-carrying air exits the aerosol provision system through the mouthpiece opening for inhalation by the user.

Usually an electric current is supplied to the heater when a user is drawing/ puffing on the device. Typically, the electric current is supplied to the heater, e.g. resistance heating element, in response to either the activation of an airflow sensor along the flow path as the user inhales/draw/puffs or in response to the activation of a button by the user. The heat generated by the heating element is used to vaporise a formulation. The released vapour mixes with air drawn through the device by the puffing consumer and forms an aerosol. Alternatively or in addition, the heating element is used to heat but typically not burn a botanical such as tobacco, to release active ingredients thereof as a vapour / aerosol.

The amount of vaporised / aerosolised payload inhaled by the user will depend at least in part on how long and how deeply the user inhales and, over a period of time, how frequently the user inhales as well. In turn, these user behaviours may be influenced by their mood.

Consequently it would be useful to provide an aerosol delivery mechanism that was more responsive to the user's mood or behaviour.

### SUMMARY OF THE INVENTION

In a first aspect, a system comprising an aerosol provision system is provided in accordance with claim 1.

In another aspect, a method of aerosol provision is provided in accordance with claim 11.

Further aspects of the invention are provided as recited in the claims.

It is to be understood that both the foregoing general summary of the disclosure and the following detailed description are exemplary, but are not restrictive, of the disclosure.

### BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:
- Figure 1 illustrates an electronic aerosol / vapour provision system (EVPS).
- Figure 2 illustrates further details of the EVPS.
- Figure 3 illustrates further details of the EVPS.
- Figure 4 illustrates further details of the EVPS.
- Figure 5 illustrates a system comprising the EVPS and a remote device.
- Figure 6 is a flowchart for a method of aerosol provision.

### DESCRIPTION OF THE EMBODIMENTS

An electronic aerosol provision system and method are disclosed. In the following description, a number of specific details are presented in order to provide a thorough understanding of the embodiments of the present disclosure. It will be apparent, however, to a person skilled in the art that these specific details need not be employed to practice embodiments of the present disclosure. Conversely, specific details known to the person skilled in the art are omitted for the purposes of clarity where appropriate.

As described above, the present disclosure relates to an aerosol provision system (e.g. a non-combustible aerosol provision system) or electronic vapour provision system (EVPS), such as an e-cigarette. Throughout the following description the term "e-cigarette" is sometimes used but this term may be used interchangeably with (electronic) aerosol/vapour provision system. Similarly the terms `vapour' and 'aerosol' are referred to equivalently herein.

Generally, the electronic vapour / aerosol provision system may be an electronic cigarette, also known as a vaping device or electronic nicotine delivery system (END), although it is noted that the presence of nicotine in the aerosolisable material is not a requirement. In some embodiments, a non-combustible aerosol provision system is a tobacco heating system, also known as a heat-not-burn system. In some embodiments, the non-combustible aerosol provision system is a hybrid system to generate aerosol using a combination of aerosolisable materials, one or a plurality of which may be heated. Each of the aerosolisable materials may be, for example, in the form of a solid, liquid or gel and may or may not contain nicotine. In some embodiments, the hybrid system comprises a liquid or gel aerosolisable material and a solid aerosolisable material. The solid aerosolisable material may comprise, for example, tobacco or a non-tobacco product. Meanwhile in some embodiments, the non-combustible aerosol provision system generates a vapour / aerosol from one or more such aerosolisable materials.

Typically, the non-combustible aerosol provision system may comprise a non-combustible aerosol provision device and an article for use with the non-combustible aerosol provision system. However, it is envisaged that articles which themselves comprise a means for powering an aerosol generating component may themselves form the non-combustible aerosol provision system. In one embodiment, the non-combustible aerosol provision device may comprise a power source and a controller. The power source may be an electric power source or an exothermic power source. In one embodiment, the exothermic power source comprises a carbon substrate which may be energised so as to distribute power in the form of heat to an aerosolisable material or heat transfer material in proximity to the exothermic power source. In one embodiment, the power source, such as an exothermic power source, is provided in the article so as to form the non-combustible aerosol provision. In one embodiment, the article for use with the non-combustible aerosol provision device may comprise an aerosolisable material.

In some embodiments, the aerosol generating component is a heater capable of interacting with the aerosolisable material so as to release one or more volatiles from the aerosolisable material to form an aerosol. In one embodiment, the aerosol generating component is capable of generating an aerosol from the aerosolisable material without heating. For example, the aerosol generating component may be capable of generating an aerosol from the aerosolisable material without applying heat thereto, for example via one or more of vibrational, mechanical, pressurisation or electrostatic means.

In some embodiments, the aerosolisable material may comprise an active material, an aerosol forming material and optionally one or more functional materials. The active material may comprise nicotine (optionally contained in tobacco or a tobacco derivative) or one or more other non-olfactory physiologically active materials. A non-olfactory physiologically active material is a material which is included in the aerosolisable material in order to achieve a physiological response other than olfactory perception. The aerosol forming material may comprise one or more of glycerine, glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate. The one or more functional materials may comprise one or more of flavours, carriers, pH regulators, stabilizers, and/or antioxidants.

In some embodiments, the article for use with the non-combustible aerosol provision device may comprise aerosolisable material or an area for receiving aerosolisable material. In one embodiment, the article for use with the non-combustible aerosol provision device may comprise a mouthpiece. The area for receiving aerosolisable material may be a storage area for storing aerosolisable material. For example, the storage area may be a reservoir. In one embodiment, the area for receiving aerosolisable material may be separate from, or combined with, an aerosol generating area.

Referring now to the drawings, wherein like reference numerals designate identical or corresponding parts throughout the several views,

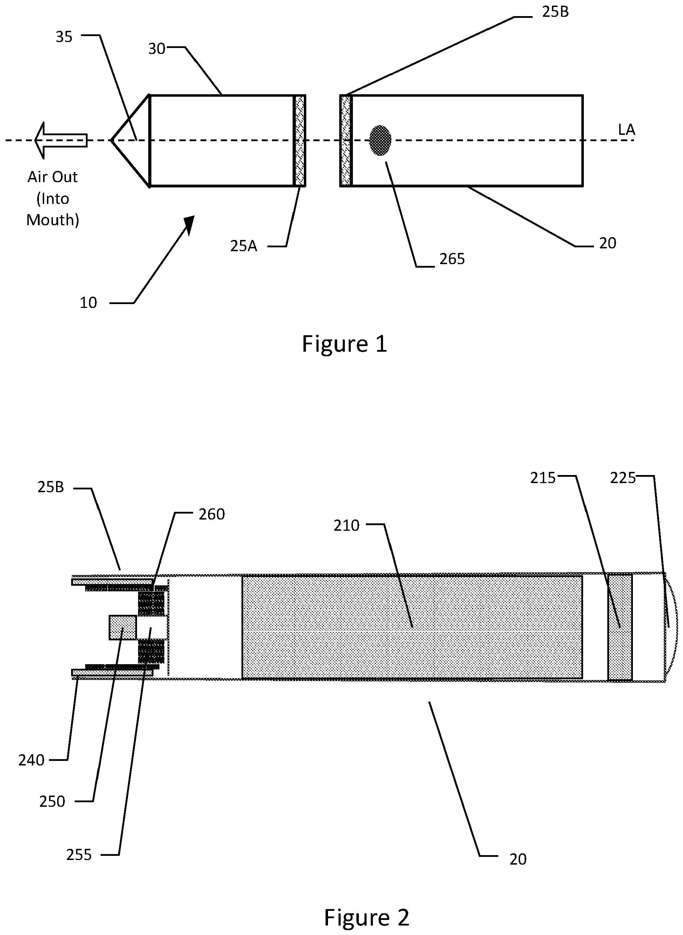

Figure 1 is a schematic diagram of an electronic vapour / aerosol provision system such as an e-cigarette 10 in accordance with some embodiments of the disclosure (not to scale). The e-cigarette has a generally cylindrical shape, extending along a longitudinal axis indicated by dashed line LA, and comprises two main components, namely a body 20 and a cartomiser 30. The cartomiser includes an internal chamber containing a reservoir of a payload such as for example a liquid comprising nicotine, a vaporiser (such as a heater), and a mouthpiece 35. References to 'nicotine' hereafter will be understood to be merely exemplary and can be substituted with any suitable active ingredient. References to `liquid' as a payload hereafter will be understood to be merely exemplary and can be substituted with any suitable payload such as botanical matter (for example tobacco that is to be heated rather than burned), or a gel comprising an active ingredient and/or flavouring. The reservoir may be a foam matrix or any other structure for retaining the liquid until such time that it is required to be delivered to the vaporiser. In the case of a liquid / flowing payload, the vaporiser is for vaporising the liquid, and the cartomiser 30 may further include a wick or similar facility to transport a small amount of liquid from the reservoir to a vaporising location on or adjacent the vaporiser. In the following, a heater is used as a specific example of a vaporiser. However, it will be appreciated that other forms of vaporiser (for example, those which utilise ultrasonic waves) could also be used and it will also be appreciated that the type of vaporiser used may also depend on the type of payload to be vaporised.

The body 20 includes a re-chargeable cell or battery to provide power to the e-cigarette 10 and a circuit board for generally controlling the e-cigarette. When the heater receives power from the battery, as controlled by the circuit board, the heater vaporises the liquid and this vapour is then inhaled by a user through the mouthpiece 35. In some specific embodiments the body is further provided with a manual activation device 265, e.g. a button, switch, or touch sensor located on the outside of the body.

The body 20 and cartomiser 30 may be detachable from one another by separating in a direction parallel to the longitudinal axis LA, as shown in Figure 1, but are joined together when the device 10 is in use by a connection, indicated schematically in Figure 1 as 25A and 25B, to provide mechanical and electrical connectivity between the body 20 and the cartomiser 30. The electrical connector 25B on the body 20 that is used to connect to the cartomiser 30 also serves as a socket for connecting a charging device (not shown) when the body 20 is detached from the cartomiser 30. The other end of the charging device may be plugged into a USB socket to recharge the cell in the body 20 of the e-cigarette 10. In other implementations, a cable may be provided for direct connection between the electrical connector 25B on the body 20 and a USB socket.

The e-cigarette 10 is provided with one or more holes (not shown in Figure 1) for air inlets. These holes connect to an air passage through the e-cigarette 10 to the mouthpiece 35. When a user inhales through the mouthpiece 35, air is drawn into this air passage through the one or more air inlet holes, which are suitably located on the outside of the e-cigarette. When the heater is activated to vaporise the nicotine from the cartridge, the airflow passes through, and combines with, the generated vapour, and this combination of airflow and generated vapour then passes out of the mouthpiece 35 to be inhaled by a user. Except in single-use devices, the cartomiser 30 may be detached from the body 20 and disposed of when the supply of liquid is exhausted (and replaced with another cartomiser if so desired).

It will be appreciated that the e-cigarette 10 shown in Figure 1 is presented by way of example, and various other implementations can be adopted. For example, in some embodiments, the cartomiser 30 is provided as two separable components, namely a cartridge comprising the liquid reservoir and mouthpiece (which can be replaced when the liquid from the reservoir is exhausted), and a vaporiser comprising a heater (which is generally retained). As another example, the charging facility may connect to an additional or alternative power source, such as a car cigarette lighter.

Figure 2 is a schematic (simplified) diagram of the body 20 of the e-cigarette 10 of Figure 1 in accordance with some embodiments of the disclosure. Figure 2 can generally be regarded as a cross-section in a plane through the longitudinal axis LA of the e-cigarette 10. Note that various components and details of the body, e.g. such as wiring and more complex shaping, have been omitted from Figure 2 for reasons of clarity.

The body 20 includes a battery or cell 210 for powering the e-cigarette 10 in response to a user activation of the device. Additionally, the body 20 includes a control unit (not shown in Figure 2), for example a chip such as an application specific integrated circuit (ASIC) or microcontroller, for controlling the e-cigarette 10. The microcontroller or ASIC includes a CPU or micro-processor. The operations of the CPU and other electronic components are generally controlled at least in part by software programs running on the CPU (or other component). Such software programs may be stored in non-volatile memory, such as ROM, which can be integrated into the microcontroller itself, or provided as a separate component. The CPU may access the ROM to load and execute individual software programs as and when required. The microcontroller also contains appropriate communications interfaces (and control software) for communicating as appropriate with other devices in the body 10.

The body 20 further includes a cap 225 to seal and protect the far (distal) end of the e-cigarette 10. Typically there is an air inlet hole provided in or adjacent to the cap 225 to allow air to enter the body 20 when a user inhales on the mouthpiece 35. The control unit or ASIC may be positioned alongside or at one end of the battery 210. In some embodiments, the ASIC is attached to a sensor unit 215 to detect an inhalation on mouthpiece 35 (or alternatively the sensor unit 215 may be provided on the ASIC itself). An air path is provided from the air inlet through the e-cigarette, past the airflow sensor 215 and the heater (in the vaporiser or cartomiser 30), to the mouthpiece 35. Thus when a user inhales on the mouthpiece of the e-cigarette, the CPU detects such inhalation based on information from the airflow sensor 215.

At the opposite end of the body 20 from the cap 225 is the connector 25B for joining the body 20 to the cartomiser 30. The connector 25B provides mechanical and electrical connectivity between the body 20 and the cartomiser 30. The connector 25B includes a body connector 240, which is metallic (silver-plated in some embodiments) to serve as one terminal for electrical connection (positive or negative) to the cartomiser 30. The connector 25B further includes an electrical contact 250 to provide a second terminal for electrical connection to the cartomiser 30 of opposite polarity to the first terminal, namely body connector 240. The electrical contact 250 is mounted on a coil spring 255. When the body20 is attached to the cartomiser 30, the connector 25A on the cartomiser 30 pushes against the electrical contact 250 in such a manner as to compress the coil spring in an axial direction, i.e. in a direction parallel to (co-aligned with) the longitudinal axis LA. In view of the resilient nature of the spring 255, this compression biases the spring 255 to expand, which has the effect of pushing the electrical contact 250 firmly against connector 25A of the cartomiser 30, thereby helping to ensure good electrical connectivity between the body 20 and the cartomiser 30. The body connector 240 and the electrical contact 250 are separated by a trestle 260, which is made of a non-conductor (such as plastic) to provide good insulation between the two electrical terminals. The trestle 260 is shaped to assist with the mutual mechanical engagement of connectors 25A and 25B.

As mentioned above, a button 265, which represents a form of manual activation device 265, may be located on the outer housing of the body 20. The button 265 may be implemented using any appropriate mechanism which is operable to be manually activated by the user - for example, as a mechanical button or switch, a capacitive or resistive touch sensor, and so on. It will also be appreciated that the manual activation device 265 may be located on the outer housing of the cartomiser 30, rather than the outer housing of the body 20, in which case, the manual activation device 265 may be attached to the ASIC via the connections 25A, 25B. The button 265 might also be located at the end of the body 20, in place of (or in addition to) cap 225.

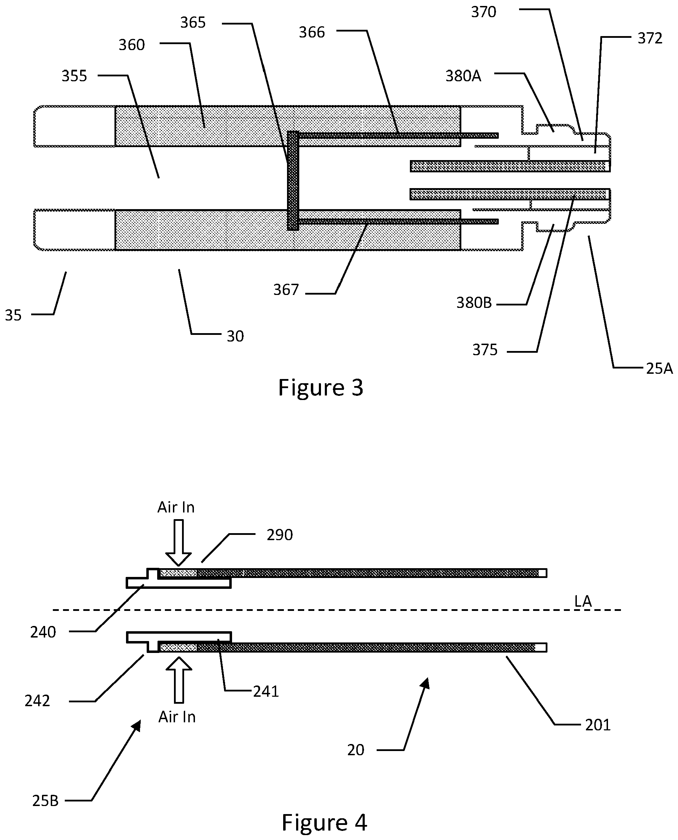

Figure 3 is a schematic diagram of the cartomiser 30 of the e-cigarette 10 of Figure 1 in accordance with some embodiments of the disclosure. Figure 3 can generally be regarded as a cross-section in a plane through the longitudinal axis LA of the e-cigarette 10. Note that various components and details of the cartomiser 30, such as wiring and more complex shaping, have been omitted from Figure 3 for reasons of clarity.

The cartomiser 30 includes an air passage 355 extending along the central (longitudinal) axis of the cartomiser 30 from the mouthpiece 35 to the connector 25A for joining the cartomiser 30 to the body 20. A reservoir of liquid 360 is provided around the air passage 335. This reservoir 360 may be implemented, for example, by providing cotton or foam soaked in liquid. The cartomiser 30 also includes a heater 365 for heating liquid from reservoir 360 to generate vapour to flow through air passage 355 and out through mouthpiece 35 in response to a user inhaling on the e-cigarette 10. The heater 365 is powered through lines 366 and 367, which are in turn connected to opposing polarities (positive and negative, or vice versa) of the battery 210 of the main body 20 via connector 25A (the details of the wiring between the power lines 366 and 367 and connector 25A are omitted from Figure 3).

The connector 25A includes an inner electrode 375, which may be silver-plated or made of some other suitable metal or conducting material. When the cartomiser 30 is connected to the body 20, the inner electrode 375 contacts the electrical contact 250 of the body 20 to provide a first electrical path between the cartomiser 30 and the body 20. In particular, as the connectors 25A and 25B are engaged, the inner electrode 375 pushes against the electrical contact 250 so as to compress the coil spring 255, thereby helping to ensure good electrical contact between the inner electrode 375 and the electrical contact 250.

The inner electrode 375 is surrounded by an insulating ring 372, which may be made of plastic, rubber, silicone, or any other suitable material. The insulating ring is surrounded by the cartomiser connector 370, which may be silver-plated or made of some other suitable metal or conducting material. When the cartomiser 30 is connected to the body 20, the cartomiser connector 370 contacts the body connector 240 of the body 20 to provide a second electrical path between the cartomiser 30 and the body 20. In other words, the inner electrode 375 and the cartomiser connector 370 serve as positive and negative terminals (or vice versa) for supplying power from the battery 210 in the body 20 to the heater 365 in the cartomiser 30 via supply lines 366 and 367 as appropriate.

The cartomiser connector 370 is provided with two lugs or tabs 380A, 380B, which extend in opposite directions away from the longitudinal axis of the e-cigarette 10. These tabs are used to provide a bayonet fitting in conjunction with the body connector 240 for connecting the cartomiser 30 to the body 20. This bayonet fitting provides a secure and robust connection between the cartomiser 30 and the body 20, so that the cartomiser and body are held in a fixed position relative to one another, with minimal wobble or flexing, and the likelihood of any accidental disconnection is very small. At the same time, the bayonet fitting provides simple and rapid connection and disconnection by an insertion followed by a rotation for connection, and a rotation (in the reverse direction) followed by withdrawal for disconnection. It will be appreciated that other embodiments may use a different form of connection between the body 20 and the cartomiser 30, such as a snap fit or a screw connection.

Figure 4 is a schematic diagram of certain details of the connector 25B at the end of the body 20 in accordance with some embodiments of the disclosure (but omitting for clarity most of the internal structure of the connector as shown in Figure 2, such as trestle 260). In particular,

Figure 4 shows the external housing 201 of the body 20, which generally has the form of a cylindrical tube. This external housing 201 may comprise, for example, an inner tube of metal with an outer covering of paper or similar. The external housing 201 may also comprise the manual activation device 265 (not shown in Figure 4) so that the manual activation device 265 is easily accessible to the user.

The body connector 240 extends from this external housing 201 of the body 20. The body connector 240 as shown in Figure 4 comprises two main portions, a shaft portion 241 in the shape of a hollow cylindrical tube, which is sized to fit just inside the external housing 201 of the body 20, and a lip portion 242 which is directed in a radially outward direction, away from the main longitudinal axis (LA) of the e-cigarette. Surrounding the shaft portion 241 of the body connector 240, where the shaft portion does not overlap with the external housing 201, is a collar or sleeve 290, which is again in a shape of a cylindrical tube. The collar 290 is retained between the lip portion 242 of the body connector 240 and the external housing 201 of the body, which together prevent movement of the collar 290 in an axial direction (i.e. parallel to axis LA). However, collar 290 is free to rotate around the shaft portion 241 (and hence also axis LA).

As mentioned above, the cap 225 is provided with an air inlet hole to allow air to flow when a user inhales on the mouthpiece 35. However, in some embodiments the majority of air that enters the device when a user inhales flows through collar 290 and body connector 240 as indicated by the two arrows in Figure 4.

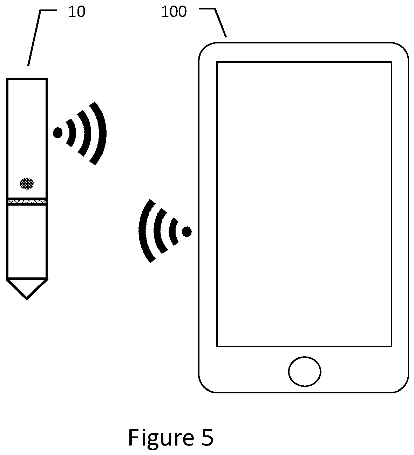

Referring again to figure 1 and also figure 5, in an embodiment of the present disclosure, an aerosol provision system, configured to generate aerosol from an aerosol generating material for user inhalation, the system, comprises a computer (not shown). As will be described later herein, the computer may be located in the electronic vapour provision system (EVPS) 10, or in a mobile device 100 such as a phone operable to communicate with the EPVS, or the functions of the computer may be shared between processors on each of these devices, and/or a remote server (not shown).

This computer is then configured (for example by suitable software instruction) to perform the following functions.
- firstly, it obtains user behaviour data relating to an interaction by a user with the aerosol provision system;
- secondly, it determines default user behaviour with respect to the interaction on the basis of the obtained user behaviour data;
- thirdly, it monitors a current interaction by the user with the aerosol provision system; and
- then, when the current user interaction deviates from the default user behaviour by a pre-determined amount, fourthly it adjusts the operational parameters of the aerosol provision system on the basis of the current user interaction.

With regards to the first function, user behaviour data may comprise any sensor data or user interface interaction data relating to usage of the aerosol provision system by the user.

Examples relating directly to inhalation based interactions include one or more of the frequency of inhalation actions, regularity / irregularity / distribution of inhalation actions, shallowness / depth / volume of inhalation, and duration of inhalation. Typically these are detected using an air pressure sensor (typically having a primary use of detecting inhalation to trigger the delivery of aerosol), and a timekeeping means such as a clock associated with the computer.

Examples relating to handling/manual interactions include one or more of motion-based behaviours, such as leaving the aerosol provision system alone (static), in a pocket or bag (typically a mixture of low-frequency motions), holding the device passively (typically again a mixture of low-frequency motions, in conjunction with touch detection), fiddling or toying with the device (typically a mixture of high-frequency motions, optionally in conjunction with touch detection, and/or repeated interaction with user interface components associated with the aerosol provision system that do not directly trigger delivery of aerosol themselves), and placing the device in the user's mouth either as a preamble to inhalation, or as a separate act. Typically these are detected by a mix of accelerometer / gyroscopic sensors, touch sensors, pressure sensors, stress sensors, UI inputs, and the like. Like the inhalation based interactions, handling/manual interactions may also be characterised by one or more of frequency of interaction, regularity/irregularity/distribution of the interaction, intensity of the interaction (for example in terms of continuous variables such as degree of motion or amount of pressure) and duration of interaction.

It will be appreciated that the computer may obtain user behaviour data relating to one type of interaction, or to two or more different types of interaction, such as from the examples above, or any other suitable type or types.

Similarly, it will be appreciated that the computer may obtain user behaviour data for a plurality of sample points within a predetermined period of time. Hence user data for each hour of the day may be collected, so that behaviour on an hour by hour basis over the course of a day may be determined. Alternatively or in addition user data for different locations may be collected, optionally with filters limiting locations to those visited multiple times, and/or where the user remains for more than a threshold period of time. It will be appreciated therefore that this can also equate to obtaining user behaviour data for a plurality of sample points within a predetermined period of time, where that period corresponds to the user's measured, typical, or average duration at a location (for example home, work, in town, etc.,). Optionally, transit may be considered a virtual location as well (in this case, the locations of the moving, for example when the user is in a car or on a train). Optionally, a user may blacklist a current location via a user interface so that it is not included in such data.

With regards to the second function, default user behaviour may be determined by the computer with respect to the interaction on the basis of the obtained user behaviour data. As noted above with regard to the first function this may optionally be determined with respect to two or more types of interaction on the basis of the obtained user behaviour data, and/or optionally be determined for a corresponding plurality of points within the predetermined period of time (e.g. for hourly slots in a day, or periods / locations corresponding one or more of work, rest, travel, etc.).

In any of these cases, default user behaviour may be determined by any suitable statistical analysis.

In a first instance, for example, where the user data relates to a continuous variable such as frequency of inhalation, volume of inhalation or duration of inhalation, then an average value for this property may be determined as the default. Optionally the variance or standard deviation of the average may also be determined as an indicator of how accurate or reliable this determined default user behaviour is (hence for example a narrow variance suggests an accurate estimate, whilst a wide variance suggests that whilst the estimate is representative, it is unlikely to be accurate for any individual instance).

Other data may be expressed using multiple continuous variables, such as values characterising the regularity of inhalation, or the distribution pattern of inhalation; for example a user may habitually inhale in a so-called 'bursting' pattern, where the user frequently puffs on the aerosol provision system for a limited period of time before pausing for an extended period of time, and then repeating this pattern. Alternatively the user may habitually inhale in the so-called `grazing' pattern, where the user puffs on the aerosol provision system less frequently than in the bursting pattern, but without extended pauses.

Such patterns may be expressed using average puff frequency data, but may be more accurately expressed with the addition of a low-frequency or aggregate puff frequency data showing patterns or their absence over a longer timeframe commensurate with the bursting pattern. Alternatively, the pattern may be expressed using average puff frequency data compiled for puffs separated by less than a threshold amount of time, together with average pause times for gaps in inhalation greater than that threshold amount of time. Alternatively or in addition, such plans may be expressed using other analyses, such as k-means clustering of puff records and/or any of the above values to classify the user behaviour as bursting, grazing or any other characteristic pattern identifiable within the data.

Other examples of 'bursting' patterns may include fiddling or toying with the aerosol provision system, which may comprise values descriptive of the fiddling or toying itself, and also the frequency and/or regularity with which this occurs. Again, alternatively or in addition classification of such behaviours may be implemented using any suitable clustering means.

In any event, for one or more types of data and/or one or more periods of time or equally locations, a statistical representation of the user's typical or default behaviour for a particular facet of their interaction with the aerosol provision system may be determined in this manner.

It will be appreciated that such a determination relates to behaviour predictable over a long period of time, for example in the order of weeks or months; hence even if a statistical model relates to an individual hour of the day, it is based on data for that hour of the day derived from multiple days, and potentially multiple weeks or even months.

Optionally, in recognition that a user can slowly change their behaviour (for example due to a change in personal circumstance, a change of work, or as part of a behaviour cessation plan), then such statistical representations of typical/default user behaviour may be rolling representations (for example based on the last *N* days, *M* weeks or O months of data), or multiple representations of the same data may be maintained; for example statistical representation of the typical/default user behaviour for a particular interaction may be based on a month's data, but a separate measure behaviour may be based on the last week's data; accordingly, if the variance in the separate measure exceeds a threshold indicative that the user's behaviour is changing, and/or if the average diverges from the longer term average, then this may indicate the need to build or start to build a new statistical representation based on more recent data.

With regards to the third function, the computer monitors a current interaction by the user with the aerosol provision system using any suitable means, typically at least a subset of the same means used to gather the user behaviour data underlying the default user behaviour previously determined by the computer.

Hence for example data relating to frequency of inhalation, volume and/or duration of inhalation may again be detected using an air pressure sensor and a timekeeping means, as discussed previously.

As part of the monitoring process, the computer may perform corresponding statistical analyses to those described previously herein with regards to the determination of typical/default user behaviour, to enable or simplify comparison.

In this case however, the statistical analysis is performed on relatively short-term user behaviour data. Hence for example whilst a facet of typical user behaviour for a given location or a given time of day may be determined using data over a number of days or weeks, the current interaction may be monitored based on behaviour in the last 5, 15, 30, 60, 90 or 120 minutes, or as appropriate based on data since the user arrived at a given location, entered a given state, or commenced a detectable activity. Meanwhile optionally some longer term user behaviours (for example seasonal trends) may be monitored as current behaviours on a daily or weekly bases.

The short-term user behaviour data, processed as appropriate if required, may then be compared with the determined typical / default / long term user behaviour data for one or more facets/types of user behaviour.

Accordingly, a further function of the computer is to compare current interactions with default user behaviour. This may be considered to be part of the monitoring function, or part of a subsequent conditional adjustment function, or a separate function in its own right.

The comparison is intended to check for when the current user interaction deviates from the default user behaviour by a pre-determined amount, or similarly when two or more of the current user interactions deviate from the default user behaviour by respective pre-determined amounts, if two or more are being monitored and compared.

If the behaviours are either time, location or user-state dependent, then similarly current interactions may be compared with default user behaviour for a current respective point within the predetermined period of time (e.g. the relevant time block, or period in which the user is in the relevant location or state).

As noted above with respect to the determination of the default user behaviour, the nature of the deviation may take any suitable form; hence for example in the case of a continuous variable it may be an absolute value deviation from an average value, or maybe a relative value deviation (e.g. a percentile difference). Meanwhile for multivariate descriptions such as for example high pass filter and low-pass filtered inhalation values together characterising bursting or grazing behaviour, then deviation from the individual variables may be assessed either separately or with respect to an overall score based on an optionally weighted combination of deviation extents. Finally again with regards to classification of behaviours, deviation may be represented by a switch in classification, or where appropriate by a change in a confidence measure in the classification provided. Other measures of deviation with appropriate to respective statistical representations of user behaviour will be apparent skilled person.

Similarly, the predetermined amount (corresponding to a threshold) may be an absolute or relative threshold, or may take account of innate variability in the underlying behaviour being measured by being a whole or fractional standard deviation from the mean in the data. Again for multivariate descriptions such thresholds may be combined using any suitable weighting, and/or any suitable logical relationship, as described later herein.

Similarly where two separate behaviours are being monitored, the respective thresholds for deviation from typical/default behaviour may be evaluated separately, or as an optionally weighted combination and/or using any suitable logical relationship, as described later herein.

Meanwhile for classifications, the threshold may be built into the classification selection mechanism, and/or may relate to a threshold confidence level for the current classification.

Where values and/or behaviours are being assessed, then as noted above these can be combined using any suitable logical relationship. For example by using the OR relationship, a subsequent adjustment response can be triggered if any single behaviour deviates from expectation by its threshold amount. Meanwhile using the AND relationship requires every behaviour to deviate from expectation by its threshold amount. Meanwhile functions such as XOR can ensure that a subsequent adjustment response is triggered if only one of several behaviours deviate from expectation.

By combining values and/or behaviours in this manner, false positives can be reduced. For example, if a user habitually grazes, but for whatever reason uses their aerosol provision system more frequently for a brief period of time, the high pass frequency values will deviate from expected norms, whilst the low pass through the valleys will deviate much less because there is not a corresponding cessation of inhalation between bursts. Hence in this case requiring a deviation to meet a threshold for high AND low frequency values may avoid a false positive detection of a change from grazing to bursting behaviour.

Similarly a user may habitually toy with their aerosol provision system when they change from grazing to bursting behaviour, for example with both being symptomatic of increased stress.

Optionally the user can logically link these behaviours through a user interface, or optionally the computer may also analyse different determined behaviours for correlations in time (for example within a suitable time period such as a common 5, 15, 30, 60, 90, or 120 minute timeframe). If the correlation is above a predetermined threshold, then optionally the computer may automatically add an AND condition between the two behaviours to limit false positives.

In any event, when the or each compared current user interaction deviates from the default user behaviour by its pre-determined amount / threshold, optionally further subject to any of the combining, weighting or logical operations described above, and the computer performs its fourth function.

The computer's fourth function is to adjust the operational parameters of the aerosol provision system on the basis of the current user interactions.

It will be appreciated that where the computer is located in a device other than the aerosol provision system itself, or where its functionality is distributed across multiple devices, optionally this function may take the form of transmitting instructions to a controller of the aerosol provision system to adjust the operational parameters of that aerosol provision system.

In a first instance, this adjustment may comprise the computer determining default operational parameters that correspond with the determined default user behaviour, and then adjusting these determined default operational parameters of the aerosol provision system responsive to the degree of deviation between the current user interaction and the default user behaviour.

Hence a small deviation outside the threshold will result in a small adjustment of the appropriate operational parameters, whilst a larger deviation outside threshold will result in a correspondingly larger adjustment of the appropriate operational parameters.

In other words, the degree of change in the function of the aerosol provision system may be linearly or nonlinearly proportional to the degree of deviation of the user's behaviour from typical/default behaviour, once this deviation has exceeded the predetermined threshold.

Any suitable operational parameters related to the user's behaviour may be adjusted.

In a first instance, these may relate to the rate of delivery of the active ingredient being aerosolised/vaporised by the aerosol provision system. Hence for example the operational parameters may relate to the operating temperature of the heater used to vaporise the payload comprising the active ingredient, or equivalently the aerosolising output of any other means used for this purpose, such as a piezoelectric vibrator. This in turn may relate to the level of power provided to the heater, or a duty cycle of the heater, or the number of heating elements activated, or similar. Other examples include altering the flow rate of air through the aerosol provision system; reducing the flow rate can increase the density of vapour and increase the amount of active ingredient delivered for a given volume of air inhaled. Further examples include changing the heating profile so that more aerosol is generated at the start of an inhalation so that for a given period of inhalation more active ingredient delivered in the first part as this is more likely to be drawn deep into the lungs and hence have the desired pharmacological effect on the user. So further examples include altering the flowrate of the payload to the heater (where this is a liquid) or otherwise increasing exposure of the payload to the heater, for example by relative positioning of some or all of the payload with respect to the heater.

Alternatively or in addition, a flavour component of the payload may be altered; for example in still increasing the amount/density of active ingredient, the amount of flavour could be increased, providing a subjective impression that more aerosol and hence implicitly more active ingredient is being delivered. This may have a beneficial placebo effect without increasing the pharmacological effect of the active ingredient.

Adjustments to operational parameters of the aerosol provision system may not be limited to, or specifically relate to, the delivery of the aerosol itself. For example a user interface the aerosol provision system (or equally a mobile device associated with it) may comprise one or more of displaying a predetermined message (for example informing the user that they are deviating from normal behaviour). For example if a user is toying with the aerosol provision system at a time when this is unusual, then this may be a good time to show a battery status light, since the user's attention is more likely to be focused on the device.

Conversely, if the user has unexpectedly switched from grazing to bursting or high-frequency consumption, which may be indicative of stress or limited available time, then the provision of UI features either on the aerosol provision system itself or an accompanying app of the user's phone may be unwelcome, as might aspects of the user interface that could be considered stressful, such as the use of noises or a more frequent, complete or responsive series of notifications via a UI. Alternatively or in addition to reducing UI outputs, alternative outputs may be provided, such as playing a calming sound, or displaying calming visual display.

With regards to indicators of stress, it will be appreciated that for a given behaviour, sometimes one type of deviation may indicate heightened stress whilst another may indicate reduced stress or calm. Hence optionally, the computer may only adjust determined default operational parameters responsive to the type of deviation, with different responses (or none at all) depending on whether the deviation (or any combination, as described previously herein) increases or decreases from a typical level, or depending upon the type of classification in the event of a change, etc.

Hence optionally the computer may classify according to a predetermined criterion whether the deviation between the current user interaction(s) and the default user behaviour(s) is indicative of increased stress; and if so, initiate a stress mitigation action.

Thus in this case the adjustments to default operational parameters are specifically intended to mitigate stress when the deviation from default behaviour indicates that stress may be a cause. The classification that a change is indicative of stress may be provided by the manufacturer (for example based on user testing or feedback gathered from a plurality of users), or by the user if they are provided with a user interface that allows them to indicate that they are stressed, for example by pressing an on-screen button on a UI of the phone app. In this case the app can associate the monitored current interactions with user stress for future classification. Optionally the app may also feed this information back to the manufacturer, to enable future manufacturer settings or updates to those settings.

Hence if one or more user behaviours are classified as indicating increased stress, then the aerosol provision system may increase relative delivery of the active ingredient where this has a known calming effect, and/or simplify the user interface of the device or an associated app or otherwise make it more calming, for example by changing a background colour, and/or changing or cancelling sounds or haptic feedback of the aerosol provision system.

Optionally an associated mobile device may directly monitor for indicators of increased stress, for example by using skin galvanic sensors and/or heart rate monitors (for example in a fitness wearable in communication with the mobile phone), or analysing the user's facial expression or voice when the user is interacting with their phone.

It will be appreciated that not all adjustments to operational parameters may be proportional; some may represent a change of a binary state or a multi value state; for example whilst reducing the volume of any sounds made by the user interface responsive to the degree of apparent divergent stress behaviour of the user is proportional, optionally the user interface may simply mute audio if the deviation from default behaviour exceeds the threshold (a binary change). Similarly, the frequency and/or complexity of notifications or UI options presented to the user, for example the phone may be reduced as the deviation from default behaviour increases. In this case, the frequency may be proportional or may instead correspond to whether or not notifications having different levels of priority are displayed. For example battery and reservoir level information may be removed from the user interface unless battery power or payload reservoir a fall below respective thresholds.

It will be appreciated that if the user behaviour indicates that they may be stressed, they may not react positively to their aerosol provision system starting to behave differently in any of the manners described previously herein.

Accordingly, the aerosol provision system or an associated mobile device may ask the user if they wish for a stress mitigation action to be initiated; this may take the form of asking the user if they wish to enter a `calm mode' or similar. Alternatively the device may provide a unique user interface notification that it is changing state, for example by issuing a calming sound or melody when changing operational parameters, and/or similarly initiating a stress mitigation action.

As noted previously herein, the computer may be located in some or all of the aerosol provision system, an associated mobile device such as a mobile phone, and a remote server accessible either directly by the aerosol provision system itself (for example via Wi-Fi^{®}) or by the mobile device on behalf of the aerosol provision system.

Hence more generally a system comprising the aerosol provision system may comprise just the aerosol provision system (in turn comprising the computer), or the aerosol provision system and a mobile device such as a mobile phone (with the computer or respective functions of the computer being in one or both devices) or the aerosol provision system and a remote server (with the computer or respective functions of the computer being in one or both devices), or the aerosol provision system, a mobile device such as a mobile phone, and a remote server, with the computer or respective functions of the computer being in one or more of these devices.

It will be appreciated therefore that the above techniques may be carried out on conventional hardware suitably adapted as applicable by software instruction or by the inclusion or substitution of dedicated hardware.

Thus the required adaptation to existing parts of a conventional equivalent device may be implemented in the form of a computer program product comprising processor implementable instructions stored on a non-transitory machine-readable medium such as a floppy disk, optical disk, hard disk, solid state disk, PROM, RAM, flash memory or any combination of these or other storage media, or realised in hardware as an ASIC (application specific integrated circuit) or an FPGA (field programmable gate array) or other configurable circuit suitable to use in adapting the conventional equivalent device. Separately, such a computer program may be transmitted via data signals on a network such as an Ethernet, a wireless network, the Internet, or any combination of these or other networks.

The software used to adapt such hardware may thus carry out a corresponding method of aerosol provision, as may any dedicated configuration of hardware.

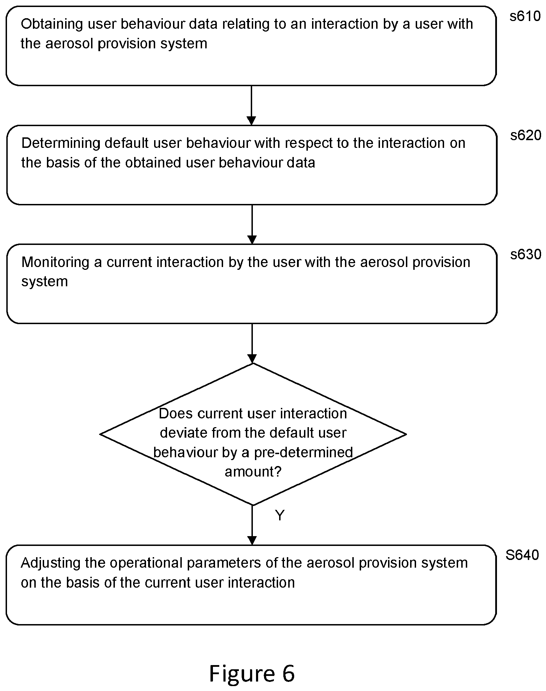

Referring now to figure 6, such a method of aerosol provision comprises:

In a first step s610, obtaining user behaviour data relating to one or interactions by a user with the aerosol provision system, as described previously herein.

In a second step s620, determining default user behaviour with respect to one or of these interaction on the basis of the obtained user behaviour data, again as described previously herein.

In a third step s630, monitoring a current interaction (or interactions) by the user with the aerosol provision system, as described previously herein.

Then as either part of the third step or the fourth step below, or as a separate intermediate step, checking whether the current user interaction deviates from the default user behaviour by a predetermined amount, as described previously herein.

Then, if the current user interaction deviates from the default user behaviour by a predetermined amount, in a fourth step s640 adjusting the operational parameters of the aerosol provision system on the basis of the current user interaction, as described previously herein.

It will be apparent to a person skilled in the art that variations in the above method corresponding to operation of the various embodiments of the method and/or apparatus as described and claimed herein are considered within the scope of the present disclosure, including but not limited to where:
- the obtaining step comprises obtaining user behaviour data relating to two or more of different types of interaction by a user with the aerosol provision system, the determining step comprises determining default user behaviour with respect to two or more types of interaction on the basis of the obtained user behaviour data, the monitoring step comprises monitoring current interactions by the user with the aerosol provision system, and when two or more of the current user interactions deviate from the default user behaviour by respective pre-determined amounts, the adjusting step comprises adjusting the operational parameters of the aerosol provision system on the basis of the current user interactions;
- the obtaining step comprises obtaining user behaviour data for a plurality of sample points within a predetermined period of time, the determining step comprises determining default user behaviour for a corresponding plurality of points within the predetermined period of time, and comparing current interactions with default user behaviour for a current respective point within the predetermined period of time;
- the determining step comprises determining default operational parameters that correspond with the determined default user behaviour, and the adjusting step comprises adjusting determined default operational parameters of the aerosol provision system responsive to the degree of deviation between the current user interaction and the default user behaviour;
- the determining step comprises determining default operational parameters that correspond with the determined default user behaviour, and the adjusting step comprises adjusting determined default operational parameters of the aerosol provision system responsive to the type of deviation between the or each current user interaction and the default user behaviour;
- classifying according to a predetermined criterion whether the deviation between the current user interaction and the default user behaviour is indicative of increased stress, and if so, initiating a stress mitigation action;
   ∘ In this case, asking the user if they wish for a stress mitigation action to be initiated, and receiving an indication from the user via a user interface;
   ∘ In this case, the stress mitigation action comprises one or more selected from the list consisting of changing the amount of an active ingredient delivered per unit volume of air inhaled, changing the amount of a flavour delivered per unit volume of air inhaled, and modifying a user interface of the aerosol provision system;
      ▪ In this latter instance, modifying a user interface of the aerosol provision system comprises one or more selected from the list consisting of displaying a predetermined message, playing a calming sound, and displaying a calming visual display; and
- the steps of the method are implemented by one or more one or more selected from the list consisting of the aerosol provision system, a remote server operable to communicate with the aerosol provision system, a mobile computing device operable to communicate with the aerosol provision system, and a remote server operable to communicate with a mobile computing device operable to communicate with the aerosol provision system.

As noted previously herein, and referring again to figure 1, the aerosol provision system may be a self-contained unit (commonly referred to as an e-cigarette, even if the device itself does not necessarily conform to the shape or dimensions of a conventional cigarette). Such an e-cigarette may comprise an airflow measuring means, a processing means and optionally one or more feedback means such as haptic, audio and/or light / display means.

Alternatively, referring to Figure 5, an aerosol provision system may comprise two components, such as an e-cigarette 10 and a mobile phone or similar device (such as a tablet) 100 operable to communicate with the e-cigarette (for example to at least receive data from the e-cigarette), for example via Bluetooth ^{®}.

The mobile phone may then comprise the processing means and one or more feedback means such as haptic, audio and/or light / display means, alternatively or in addition to those of the e-cigarette.

Optionally an aerosol provision system may comprise an e-cigarette 10 operable to communicate with a mobile phone 100, in which the mobile phone stores one or more parameters or other data (such as data characteristic of one or more aspects of usage by the user) for the aerosol provision system, and receives such parameters/data from the e-cigarette. The phone may then optionally perform processing on such parameters/data and either return processed data and/or instructions to the aerosol provision system, display a result to the user (or perform another action) or forward processed and/or unprocessed parameters/data on to a remote server.

Optionally the mobile phone or the aerosol provision system itself may be operable to wirelessly access data associated with an account of the user at such a remote server.

In a variant embodiment of the disclosure, a first aerosol provision system of a user may communicate some or all of its user settings to another aerosol provision system. The user settings may comprise settings related to an implementation of the above disclosed methods, such as data characteristic of user behaviour, and/or data relating to modification of the aerosol provision system operation.

Such data may be relayed between devices either directly (e.g. via a Bluetooth^{®} or near-field communication) or via one or more intermediary devices, such as a mobile phone owned by the user of the two devices or a server on which the user has an account.

In this way, a user may easily share the data from one device to another, for example if the user has two aerosol provision system devices, or if the user wishes to replace one aerosol provision system device with another without losing accumulated personalisation data.

Optionally in this embodiment, where the second aerosol provision system differs in type from the first aerosol provision system (for example by having a different default power level, or heating efficiency), then a conversion factor or look-up table for converting operational parameters from the first aerosol provision system to the second aerosol provision system may be employed. This may be provided in software or firmware of the second aerosol provision system, and identify the first EVPS aerosol provision system and hence the appropriate conversions when making direct communication (or where data is relayed without change via an intermediary such as a phone). Alternatively or in addition an app on the phone may provide the conversion, optionally downloading the relevant conversions in response to the identity of the first and second aerosol provision system. Again, alternatively or in addition a remote server may provide the conversion, in response to the identity of the first and second aerosol provision system as associated with a user's account.

The foregoing discussion discloses and describes merely exemplary embodiments of the present disclosure. As will be understood by those skilled in the art, the present disclosure may be embodied in other specific forms without departing from the essential characteristics thereof. Accordingly, the disclosure of the present disclosure is intended to be illustrative, but not limiting of the scope of the disclosure, as well as other claims. The disclosure, including any readily discernible variants of the teachings herein, defines, in part, the scope of the foregoing claim terminology such that no inventive subject matter is dedicated to the public.

### REPRESENTATIVE FEATURES

1. A system comprising an aerosol provision system configured to generate aerosol from an aerosol generating material for user inhalation, the system comprising a computer configured to:
   a. obtain user behaviour data relating to an interaction by a user with the aerosol provision system;
   b. determine default user behaviour with respect to the interaction on the basis of the obtained user behaviour data;
   c. monitor a current interaction by the user with the aerosol provision system; and when the current user interaction deviates from the default user behaviour by a pre-determined amount, adjust the operational parameters of the aerosol provision system on the basis of the current user interaction.
2. A system according to clause 1, in which the computer is configured to:
   obtain user behaviour data relating to two or more of different types of interaction by a user with the aerosol provision system;
   determine default user behaviour with respect to two or more types of interaction on the basis of the obtained user behaviour data;
   monitor current interactions by the user with the aerosol provision system; and
   when two or more of the current user interactions deviate from the default user behaviour by respective pre-determined amounts, adjust the operational parameters of the aerosol provision system on the basis of the current user interactions.
3. The system of clause 1 or clause 2, in which the computer is configured to:
   obtain user behaviour data for a plurality of sample points within a predetermined period of time;
   determine default user behaviour for a corresponding plurality of points within the predetermined period of time; and
   compare current interactions with default user behaviour for a current respective point within the predetermined period of time.
4. The system of any one of the preceding clauses, in which the computer is configured to:
   determine default operational parameters that correspond with the determined default user behaviour; and
   adjust determined default operational parameters of the aerosol provision system responsive to the degree of deviation between the current user interaction and the default user behaviour.
5. The system of any one of the preceding clauses, in which in which the computer is configured to:
   determine default operational parameters that correspond with the determined default user behaviour; and
   adjust determined default operational parameters of the aerosol provision system responsive to the type of deviation between the or each current user interaction and the default user behaviour.
6. The system of clause 5, in which the computer is configured to:
   classify according to a predetermined criterion whether the deviation between the current user interaction and the default user behaviour is indicative of increased stress; and if so,
   initiate a stress mitigation action.
7. The system of clause 6, in which the computer is configured to:
   ask the user if they wish for a stress mitigation action to be initiated; and
   receive an indication from the user via a user interface.
8. The system of clause 6 or 7, in which the stress mitigation action comprises one or more selected from the list consisting of:
   i. changing the amount of an active ingredient delivered per unit volume of air inhaled;
   ii. changing the amount of a flavour delivered per unit volume of air inhaled; and
   iii. modifying a user interface of the aerosol provision system.
9. The system of clause 8, in which modifying a user interface of the aerosol provision system comprises one or more selected from the list consisting of:
   i. displaying a predetermined message;
   ii. playing a calming sound; and
   iii. displaying a calming visual display
10. The system of any preceding clause, in which the operations of the computer are located within one or more selected from the list consisting of:
   i. the aerosol provision system;
   ii. a remote server operable to communicate with the aerosol provision system;
   iii. a mobile computing device operable to communicate with the aerosol provision system; and
   iv. a remote server operable to communicate with a mobile computing device operable to communicate with the aerosol provision system.
11. A method of aerosol provision comprising:
   obtaining user behaviour data relating to an interaction by a user with the aerosol provision system;
   determining default user behaviour with respect to the interaction on the basis of the obtained user behaviour data;
   monitoring a current interaction by the user with the aerosol provision system; and
   when the current user interaction deviates from the default user behaviour by a pre-determined amount, adjusting the operational parameters of the aerosol provision system on the basis of the current user interaction.
12. The method of clause 11, in which:
   the obtaining step comprises obtaining user behaviour data relating to two or more of different types of interaction by a user with the aerosol provision system;
   the determining step comprises determining default user behaviour with respect to two or more types of interaction on the basis of the obtained user behaviour data;
   the monitoring step comprises monitoring current interactions by the user with the aerosol provision system; and
   when two or more of the current user interactions deviate from the default user behaviour by respective pre-determined amounts, the adjusting step comprises adjusting the operational parameters of the aerosol provision system on the basis of the current user interactions.
13. The method of clause 11 or clause 12, in which:
   the obtaining step comprises obtaining user behaviour data for a plurality of sample points within a predetermined period of time;
   the determining step comprises determining default user behaviour for a corresponding plurality of points within the predetermined period of time; and
   comparing current interactions with default user behaviour for a current respective point within the predetermined period of time.
14. The method of any one of clauses 11 to 13, in which:
   the determining step comprises determining default operational parameters that correspond with the determined default user behaviour; and
   the adjusting step comprises adjusting determined default operational parameters of the aerosol provision system responsive to the degree of deviation between the current user interaction and the default user behaviour.
15. The method of any one of clauses 11 to 14, in which:
   the determining step comprises determining default operational parameters that correspond with the determined default user behaviour; and
   the adjusting step comprises adjusting determined default operational parameters of the aerosol provision system responsive to the type of deviation between the or each current user interaction and the default user behaviour.
16. The method of clause 15, comprising the steps of:
   classifying according to a predetermined criterion whether the deviation between the current user interaction and the default user behaviour is indicative of increased stress; and if so,
   initiating a stress mitigation action.
17. The method of clause 16, comprising the steps of:
   asking the user if they wish for a stress mitigation action to be initiated; and
   receiving an indication from the user via a user interface.
18. The method of clause 16 or clause 17, in which the stress mitigation action comprises one or more selected from the list consisting of:
   i. changing the amount of an active ingredient delivered per unit volume of air inhaled;
   ii. changing the amount of a flavour delivered per unit volume of air inhaled; and
   iii. modifying a user interface of the aerosol provision system.
19. The method of clause 18, in which modifying a user interface of the aerosol provision system comprises one or more selected from the list consisting of:
   i. displaying a predetermined message;
   ii. playing a calming sound; and
   iii. displaying a calming visual display
20. The method of any one of clauses 11 to 19, in which the steps of the method are implemented by one or more one or more selected from the list consisting of:
   i. the aerosol provision system;
   ii. a remote server operable to communicate with the aerosol provision system;
   iii. a mobile computing device operable to communicate with the aerosol provision system; and
   iv. a remote server operable to communicate with a mobile computing device operable to communicate with the aerosol provision system.
21. A computer program comprising computer executable instructions adapted to cause a computer system to perform the method of any one of method clauses 11 to 20.

This divisional application is divided from parent application no. EP20780289.3 (EP4044847) filed at the EPO on 17 September 2020. This divisional application comprises the full content of the earlier parent application as filed, including the description, claims, abstract, any drawings and any sequence listing. Nevertheless, protection may be sought for any features disclosed herein in combination with any features disclosed in the earlier parent application as filed, which is incorporated herein by reference.

## Claims

1. A system for controlling an aerosol provision system configured to generate aerosol from an aerosol generating material for user inhalation, the system comprising a computer configured to:
a. monitor a current inhalation based interaction by the user with the aerosol provision system;
b. compare the current interaction with determined default user behaviour, the default user behaviour comprising a rolling representation of inhalation based interactions by the user, and
c. when the current user interaction deviates from the default user behaviour, adjust an operational parameter of the aerosol provision system on the basis of the current user interaction.

2. The system of claim 1, wherein the default user behaviour comprises a rolling representation of frequency, regularity, irregularity, distribution, shallowness, depth, volume and/or duration of inhalation based interactions by the user.

3. The system of claim 1 or 2, wherein the computer is configured to adjust the rate of delivery of the active ingredient, the operating temperature of the heater, the level of power provided to the heater, a duty cycle of the heater and/or the number of heating elements activated.

4. The system of any preceding claim, wherein the computer is configured to, when the current user interaction deviates from the default user behaviour by a pre-determined amount, adjust the operational parameter of the aerosol provision system.

5. The system of claim 4, wherein the pre-determined amount is an absolute or relative threshold, or a whole or fractional standard deviation from a mean.

6. The system of any preceding claim, wherein the rolling representation is based on the last *N* days, *M* weeks or *O* months of data.

7. The system of any preceding claim, wherein the computer is configured to:
a. obtain user behaviour data relating to an interaction by a user with the aerosol provision system; and
b. determine the default user behaviour with respect to the interaction on the basis of the obtained user behaviour data.

8. A system according to claim 7, in which the computer is configured to:
obtain user behaviour data relating to two or more of different types of interaction by a user with the aerosol provision system;
determine default user behaviour with respect to two or more types of interaction on the basis of the obtained user behaviour data;
monitor current interactions by the user with the aerosol provision system; and
when two or more of the current user interactions deviate from the default user behaviour by respective pre-determined amounts, adjust the operational parameters of the aerosol provision system on the basis of the current user interactions.

9. The system of claim 7 or 8, in which the computer is configured to:
obtain user behaviour data for a plurality of sample points within a predetermined period of time;
determine default user behaviour for a corresponding plurality of points within the predetermined period of time; and
compare current interactions with default user behaviour for a current respective point within the predetermined period of time.

10. The system of any preceding claim, in which the computer is configured to:
determine default operational parameters that correspond with the determined default user behaviour; and
adjust determined default operational parameters of the aerosol provision system responsive to the degree or type of deviation between the or each current user interaction and the default user behaviour.

11. The system of claim 10, in which the computer is configured to:
classify according to a predetermined criterion whether the deviation between the current user interaction and the default user behaviour is indicative of increased stress; and if so,
initiate a stress mitigation action.

12. The system of claim 11, in which the stress mitigation action comprises one or more selected from the list consisting of:
i. changing the amount of an active ingredient delivered per unit volume of air inhaled;
ii. changing the amount of a flavour delivered per unit volume of air inhaled; and
iii. modifying a user interface of the aerosol provision system.

13. A method of controlling an aerosol provision, comprising:
monitoring a current inhalation based interaction by the user with the aerosol provision system;
comparing the current interaction with determined default user behaviour, the default user behaviour comprising a rolling representation of inhalation based interactions by the user, and
when the current user interaction deviates from the default user behaviour, adjusting an operational parameter of the aerosol provision system on the basis of the current user interaction.

14. The method of claim 13, further comprising:
obtaining user behaviour data relating to an interaction by a user with the aerosol provision system; and
determining the default user behaviour with respect to the interaction on the basis of the obtained user behaviour data.

15. A computer program comprising computer executable instructions adapted to cause a computer system to perform the method of any of method claims 13 to 14.
